# EUROPEAN PATENT APPLICATION

(11) **EP 3 275 875 A1**
(43) Date of publication of application: **31.01.2018**
(21) Application number: 16181775.4
(22) Date of filing: 28.07.2016
(51) Int. Cl.: C07D 309/08, C07D 317/34, C07C 69/675, C07C 69/708, C07C 69/734, C07C 59/58

(54) **DIOXOLANONE INTERMEDIATE USEFUL IN THE SYNTHESIS OF HOMOHARRINGTONINE**

(71) Applicant: INDENA S.p.A., 20139 Milano (IT)
(72) Inventor: ALLEGRINI, Pietro, 20139 Milano (IT); CATTANEO, Cristian, 20139 Milano (IT); CICERI, Daniele, 20139 Milano (IT); GAMBINI, Andrea, 20139 Milano (IT); PETERLONGO, Federico, 20139 Milano (IT); CERISOLI, Lucia, 70125 Bari (IT); LOMBARDO, Marco, 70125 Bari (IT)
(74) Representative: Bianchetti Bracco Minoja S.r.l.

(57) **Abstract**

The invention discloses 2-((2*R*,4*R*)-4-(4-(benzyloxy)- 4-methylpent-2-yn-1-yl)-2-(tert-butyl) -5-oxo-1,3-dioxolan-4-yl)acetic acid of formula (XIX), a process for its preparation and processes for its conversion into enantiomerically pure (*R*)-2-(2-methoxy-2-oxoethyl)-6,6-dimethyltetrahydro-2*H-*pyran-2-carboxylic acid of formula (I), a molecule useful for the synthesis of Homoharringtonine.

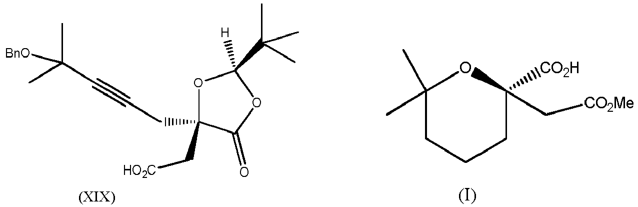

## Description

The present invention concerns a dioxolanone derivative, a process for its preparation and processes for its conversion into enantiomerically pure (*R*)-2-(2-methoxy-2-oxoethyl)-6,6-dimethyltetrahydro-2*H*-pyran-2-carboxylic acid, a molecule useful for the synthesis of Homoharringtonine.

### BACKGROUND OF THE INVENTION

(*R*)-2-(2-methoxy-2-oxoethyl)-6,6-dimethyltetrahydro-2*H-*pyran-2-carboxylic acid (I) 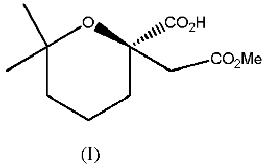 is a key intermediate in the synthesis of Homoharringtonine (II), an alkaloid used in the treatment of myeloid leukemia.

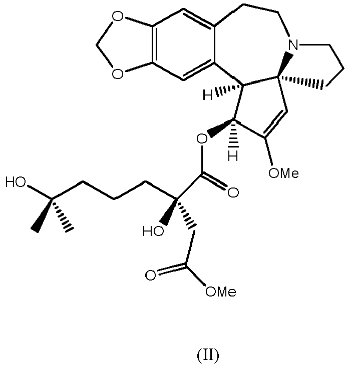

In fact, as described in US 6,831,180, the acid of formula (I) can be conveniently coupled in the presence of condensing agents with cephalotaxine (III) to provide compound (IV) which can be further manipulated to give Homoharringtonine (II).

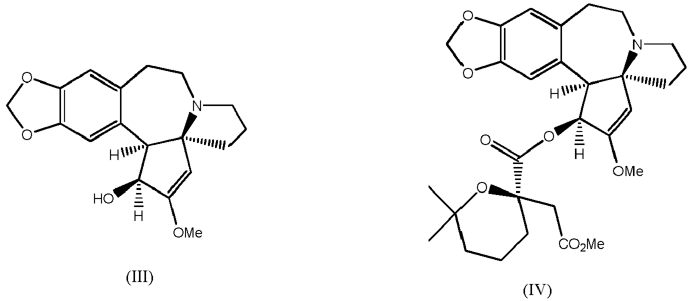

In the same prior art document, the acid of formula (I) is prepared according to the following steps:
a) Reaction of the Grignard reagent of 1-bromo-4-methyl-3-pentene (V) with diethyloxalate to obtain ethyl-2-oxo-6-methyl-5-heptenoate (VI); 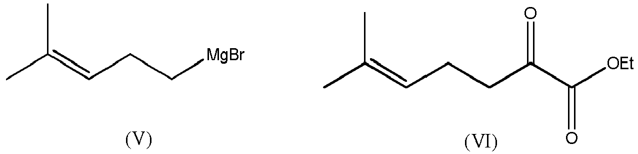
b) Reaction of compound (VI) with the enolate of methyl acetate to obtain ethyl-2-methoxycarbonylmethyl-2-hydroxy-6-methyl-5-heptenoate (VII); 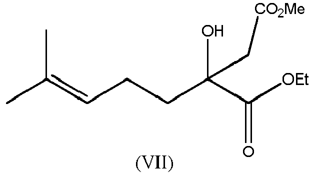
c) Hydrolysis of compound (VII) to obtain 2-carboxymethyl-2-hydroxy-6-methyl-5-heptenoic acid (VIII); 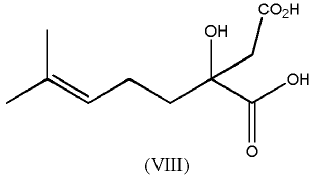
d) Cyclisation of compound (VIII) in the presence of acid to obtain 2-(carboxymethyl)-6,6-dimethyltetrahydro-2*H*-pyran-2-carboxylic acid (IX); 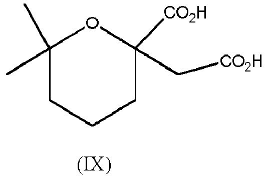
e) Monoesterification of compound (IX) to racemic 2-(2-methoxy-2-oxoethyl)-6,6-dimethyltetrahydro-2*H*-pyran-2-carboxylic acid (X); 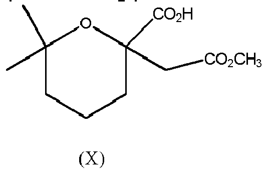
f) Enantiomeric resolution of racemate (X) to provide enantiomerically pure (*R*)-2-(2-methoxy-2-oxoethyl)-6,6-dimethyltetrahydro-2*H*-pyran-2-carboxylic acid (I).

The resolution strategy (step f) involves esterification of (X) with (-) quinine (Q-OH), followed by separation of the resulting diastereomeric mixture by chromatographic techniques.

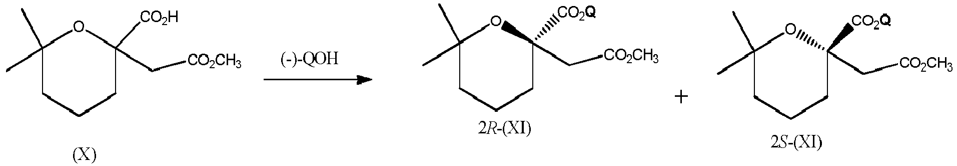

Selective removal of quinine (Q-OH) by hydrogenolysis of 2*R*-(XI) affords pure (*R*)-2-(2-methoxy-2-oxoethyl)-6,6-dimethyltetrahydro-2*H*-pyran-2-carboxylic acid (I).

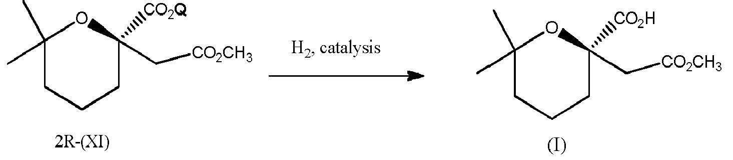

This way of producing enantiomerically pure (*R*)-2-(2-methoxy-2-oxoethyl)-6,6-dimethyltetrahydro-2*H*-pyran-2-carboxylic acid (I) is extremely inefficient since it requires a chromatographic separation, which is not an industrial purification technique, and it has a maximum of 50% yield, being the wrong enantiomer of no use for the production of Homoharringtonine (II).

WO 2007/009953 discloses a procedure for an improved purification of racemic (X), still requiring an enantiomeric resolution step.

In Eur.J.Org.Chem. 2005, 2695-2972 Lutz F. Tietze reports the use of (+) malic acid for the synthesis of enantiomerically pure (*R*)-2-hydroxy-2-(4-hydroxy-4-methylpentyl)succinic acid (XII), the diacid form of the side chain of Homoharringtonine.

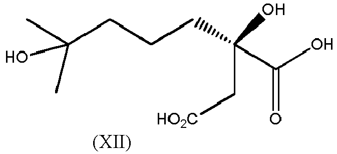

In the same prior document (XII) is prepared according to the following steps:
a) Reaction of (+) malic acid with pivalaldehyde in the presence of catalytic amounts of p-toluenesulfonic acid and sulfuric acid to produce 2-((2*R*,4*R*)-2-(tert-butyl)-5-oxo-1,3-dioxolan-4-yl)acetic acid (XIII); 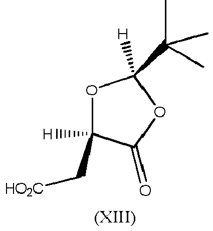
b) Reaction of (XIII) with 1-bromo-4-methyl-2-pentene (XIV) to produce 2-((2*R*,4*R*)-2-(tert-butyl)-4-((*E*)-4-methylpent-2-en-1-yl)-5-oxo-1,3-dioxolan-4-yl)acetic acid (XV); 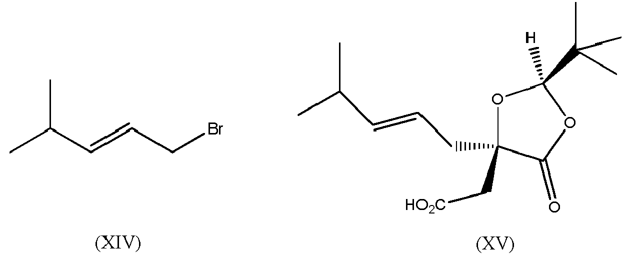
c) Allylic oxidation of (XV) with t-butyl hydroperoxide in the presence of catalytic amount of SeO₂ to produce 2-((2R,4R)-2-(tert-butyl)-4-((E)-4-hydroxy-4-methylpent-2-en-1-yl)-5-oxo-1,3-dioxolan-4-yl)acetic acid (XVI); 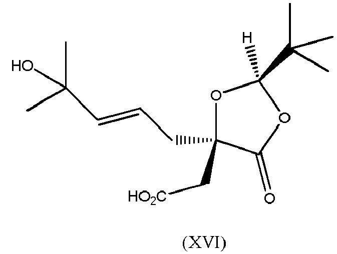
d) Catalytic hydrogenation of (XVI) to reduce the olefinic bond and producing 2-((2*R*,4*R*)-2-(tert-butyl)-4-(4-hydroxy-4-methylpentyl)-5-oxo-1,3-dioxolan-4-yl)acetic acid (XVII); 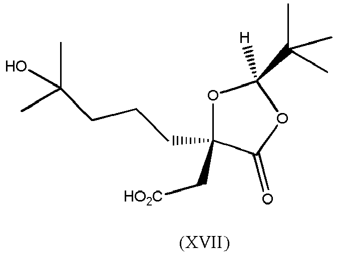
e) Reaction of (XVII) with 50 % sulfuric acid to produce enantiomerically pure (*R*)-2-hydroxy-2-(4-hydroxy-4-methylpentyl)succinic acid (XII).

(*R*)-2-hydroxy-2-(4-hydroxy-4-methylpentyl)succinic acid (XII) as such is however unsuitable for the preparation of Homoharringtonine (II) since it still requires to be monoesterified on the primary carboxylic group.

Even if not reported in the same prior art document, it can be envisioned that a simple esterification with methanol and Lewis acids would produce homoharringtonic acid (XVIII).

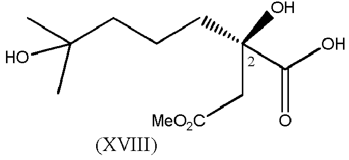

However, in spite of that, it is known from the work of Robin (Tetrahedron letters 40 (1999) 2391-2934) that, due to steric hindrance of the carboxyl group in position 2, the esterification of homoharringtonic acid (XVIII) with cephalotaxine (III) fails. Furthermore, hydrogenation of allylic alcohols, as in step d), suffers from hydrogenolysis of the C-O bond, as reported by H.O. House (Modern Synthetic Reaction, W.A. Benjamin,Inc. 1965) decreasing the reaction yield of the end product.

Therefore, there is still the need for an improved process for the preparation of an enantiomerically pure molecule, such as (I), capable of coupling efficiently with cephalotaxine (III).

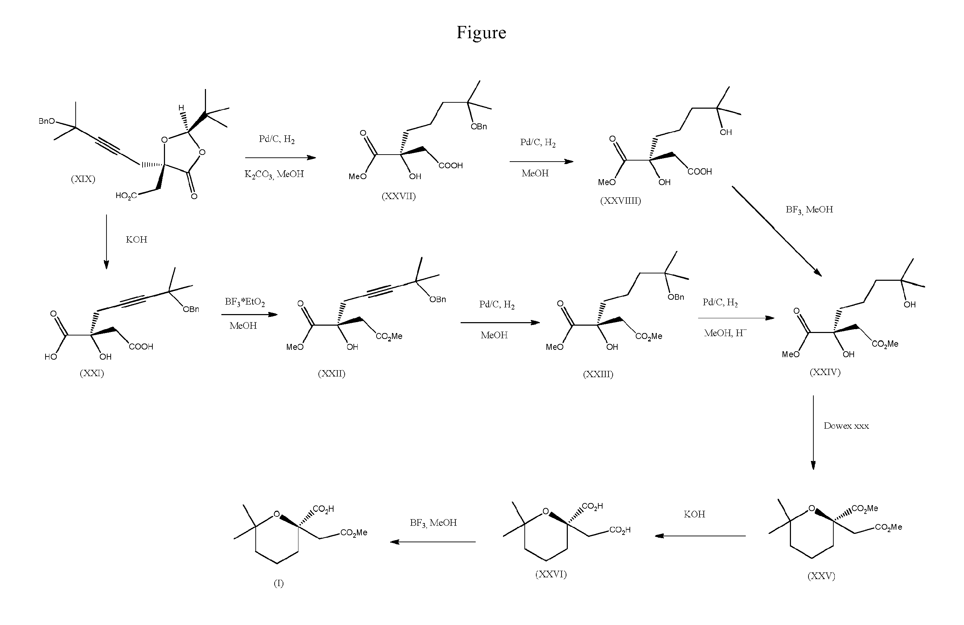

### DESCRIPTION OF THE FIGURE

The Figure shows a synthetic scheme of a preferred embodiment of the process according to the present invention.

### SUMMARY OF THE INVENTION

The applicant has now surprisingly found that a dioxolanone derivative, 2-((2R,4R)-4-(4-(benzyloxy)- 4-methylpent-2-yn-1-yl)-2-(tert-butyl) -5-oxo-1,3-dioxolan-4-yl)acetic acid of formula (XIX), 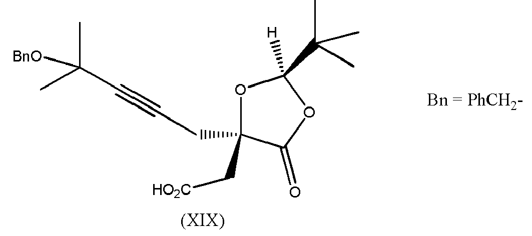 is useful for the preparation of (R)-2-(2-methoxy-2-oxoethyl)-6,6-dimethyltetrahydro-2*H*-pyran-2-carboxylic acid (I). In a further aspect of the same invention a process for the preparation of (XIX) and processes for its conversion to (I) are disclosed. The subject of the present patent overcomes the drawbacks of the above-mentioned prior arts documents.

### DETAILED DESCRIPTION OF THE INVENTION

2-((2R,4R)-4-(4-(benzyloxy)- 4-methylpent-2-yn-1-yl)-2-(tert-butyl) -5-oxo-1,3-dioxolan-4-yl)acetic acid (XIX) can be prepared by reaction of 2-((2*R*,4*R*)-2-(tert-butyl)-5-oxo-1,3-dioxolan-4-yl)acetic acid (XIII) with 5-iodo-2-methyl-pent-3-yn-2-ol benzyl ether (XX) in the presence of a base.

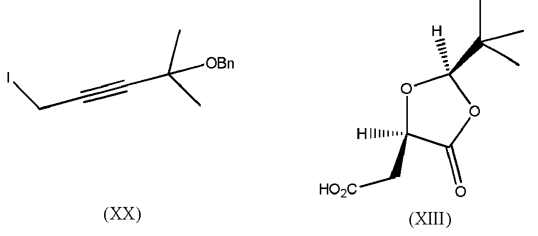

In one embodiment, the compound of formula (XIII) is reacted first with a base and then the product of such reaction is reacted with the compound of formula (XX). In a preferred embodiment, the base is a strong non-nucleophilic base, such as potassium-, lithium- or sodium-hexamethyldisilazane, or lithium diisopropylamide.

In another embodiment, the base is added in the presence of both compound (XIII) and compound (XX).

Compound (XX) and compound (XIII) can be conveniently prepared as reported in US 6,387,901 and Eur.J. Org. Chem. 2005, 2695-2972 respectively.

Compound (XIX) can be further manipulated to provide compound (I) according to the following steps:
a) Hydrolysis of 2-((2R,4R)-4-(4-(benzyloxy)- 4-methylpent-2-yn-1-yl)-2-(tert-butyl) -5-oxo-1,3-dioxolan-4-yl)acetic acid (XIX) to produce (R)-2-(4-(benzyloxy)-4-methylpent-2-yn-1-yl)-2-hydroxysuccinic acid (XXI); 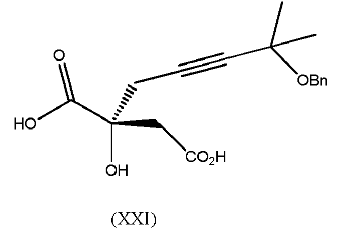
b) Esterification of (*R*)-2-(4-(benzyloxy)-4-methylpent-2-yn-1-yl)-2-hydroxysuccinic acid (XXI) to produce (R)-dimethyl 2-(4-(benzyloxy)-4-methylpent-2-yn-1-yl)-2-hydroxysuccinate (XXII); 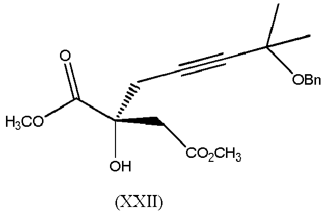
c) Hydrogenation in slightly basic conditions of (R)-dimethyl 2-(4-(benzyloxy)-4-methylpent-2-yn-1-yl)-2-hydroxysuccinate (XXII) to produce (R)-dimethyl 2-(4-(benzyloxy)-4-methylpentyl)-2-hydroxysuccinate (XXIII); 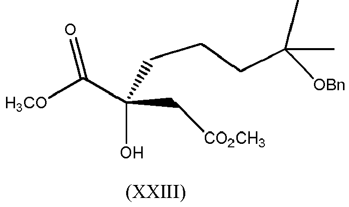
d) Hydrogenation in slightly acidic conditions of (R)-dimethyl 2-(4-(benzyloxy)-4-methylpentyl)-2-hydroxysuccinate (XXIII) to produce (R)-dimethyl 2-(4-hydroxy-4-methylpentyl)-2-hydroxysuccinate (XXIV); 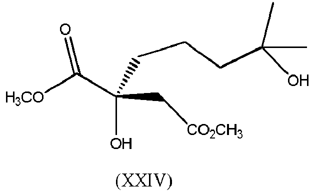
e) Cyclisation in acidic conditions of (R)-dimethyl 2-(4-hydroxy-4-methylpentyl)-2-hydroxysuccinate (XXIV) to produce (R)-methyl 2-(2-methoxy-2-oxoethyl)-6,6-dimethyltetrahydro-2*H*-pyran-2-carboxylate (XXV); 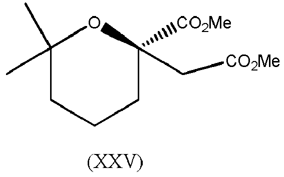
f) Hydrolysis of (*R*)-methyl 2-(2-methoxy-2-oxoethyl)-6,6-dimethyltetrahydro-2*H-*pyran-2-carboxylate (XXV) to produce (*R*)-2-(carboxymethyl)-6,6-dimethyltetrahydro-2*H*-pyran-2-carboxylic acid (XXVI); 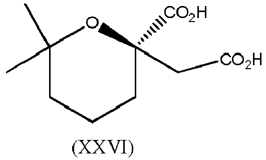
g) Selective monoesterification of (*R*)-2-(carboxymethyl)-6,6-dimethyltetrahydro-2*H*-pyran-2-carboxylic acid (XXVI) to produce (*R*)-2-(2-methoxy-2-oxoethyl)-6,6-dimethyltetrahydro-2*H*-pyran-2-carboxylic acid (I).

According to a preferred embodiment of the invention step a) is carried out with an inorganic base, preferably potassium hydroxide, in alcoholic media, preferably methanol. Step b) is carried out in methanol in the presence of acids, preferably Lewis acids, preferably BF₃. Step c) is carried out with hydrogen and an hydrogenation catalyst, preferably Pd/C, in suitable solvent, preferably methanol containing potassium carbonate. Step d) is performed with hydrogen and a hydrogenation catalyst, preferably Pd/C, in methanol containing catalytic amount of acids, preferably acetic acid. Step e) is carried out in dichloromethane in the presence of acids, preferably a strong acidic cation exchange resin. Step f) is carried out in the presence of an organic or inorganic base, preferably potassium hydroxide in methanol. Step g) is performed in methanol in the presence of acids, preferably Lewis acids, preferably BF₃.

Alternatively compound (XIX) can be converted into (I) according to the following steps:
a) Hydrogenation of (XIX) in basic conditions in the presence of methanol to provide (*R*)-7-(benzyloxy)-3-hydroxy-3-(methoxycarbonyl)-7-methyloctanoic acid (XXVII); 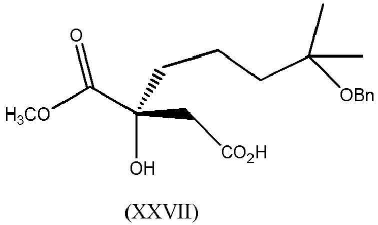
b) Hydrogenation of (R)-7-(benzyloxy)-3-hydroxy-3-(methoxycarbonyl)-7-methyloctanoic acid (XXVII) to yield (R)-3,7-dihydroxy-3-(methoxycarbonyl)-7-methyloctanoic acid (XXVIII); 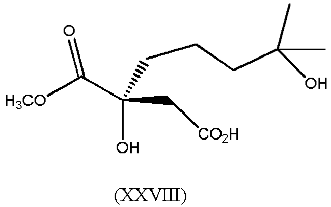
c) Esterification of (*R*)-3,7-dihydroxy-3-(methoxycarbonyl)-7-methyloctanoic acid (XXVIII) with methanol to yield (*R*)-dimethyl 2-(4-hydroxy-4-methylpentyl)-2-hydroxysuccinate (XXIV); 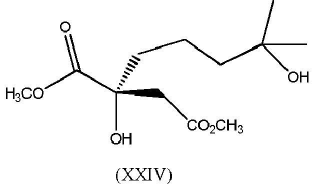
d) Cyclisation in acidic conditions of (*R*)-dimethyl 2-(4-hydroxy-4-methylpentyl)-2-hydroxysuccinate (XXIV) to produce (R)-methyl 2-(2-methoxy-2-oxoethyl)-6,6-dimethyltetrahydro-2*H*-pyran-2-carboxylate (XXV); 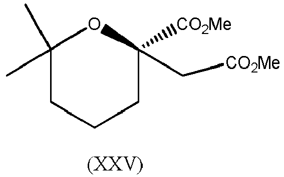
e) Hydrolysis of (*R*)-methyl 2-(2-methoxy-2-oxoethyl)-6,6-dimethyltetrahydro-2*H-*pyran-2-carboxylate (XXV) to produce (*R*)-2-(carboxymethyl)-6,6-dimethyltetrahydro-2*H*-pyran-2-carboxylic acid (XXVI); 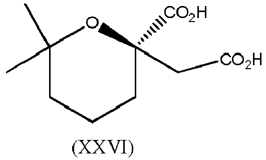
f) Selective esterification of (*R*)-2-(carboxymethyl)-6,6-dimethyltetrahydro-2*H*-pyran-2-carboxylic acid (XXVI) to produce (*R*)-2-(2-methoxy-2-oxoethyl)-6,6-dimethyltetrahydro-2*H*-pyran-2-carboxylic acid (I).

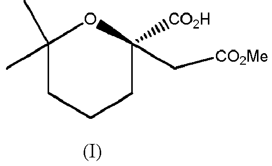

According to a preferred embodiment of the invention step a) is carried out in the presence of an hydrogenation catalyst, preferably Pd/C, hydrogen and an inorganic base, preferably potassium carbonate. Step b) is carried out in methanol in the presence of an hydrogenation catalyst, preferably Pd/C and hydrogen. Step c) is carried out in methanol in the presence of Lewis acids, preferably BF₃. Step d) in carried out in dichloromethane in the presence of acids, preferably a strong acidic cation exchange resin. Step e) is carried out in the presence of an organic or inorganic base, preferably potassium hydroxide in methanol. Step f) is carried out in methanol in the presence of acids, preferably Lewis acids, preferably BF₃.

Alternatively, the above steps b) and c) can be combined in a single step performing the hydrogenation of (R)-7-(benzyloxy)-3-hydroxy-3-(methoxycarbonyl)-7-methyloctanoic acid (XXVII) in methanol in the presence of an hydrogenation catalyst, preferably Pd/C and hydrogen under slightly acid conditions, to yield (*R*)-dimethyl 2-(4-hydroxy-4-methylpentyl)-2-hydroxysuccinate (XXIV).

The use of 2-((2*R*,4*R*)-4-(4-(benzyloxy)- 4-methylpent-2-yn-1-yl)-2-(tert-butyl) -5-oxo-1,3-dioxolan-4-yl)acetic acid (XIX), the process for its preparation and the methods of its transformation into (*R*)-2-(2-methoxy-2-oxoethyl)-6,6-dimethyltetrahydro-2*H*-pyran-2-carboxylic acid (I) are particularly advantageous because they allow the preparation of enantiomerically pure compound (I) without resorting to an enantiomeric resolution as reported in US 6,831,180. It is even more advantageous over the prior art cited in Eur.J.Org.Chem. 2005, 2695-2972 since the process of the applicant produces a molecule suitable for the coupling with cephalotaxine. Furthermore the hydrogenation of the triple bond of molecule (XXII) or alternatively (XIX) occurs when the hydroxy groups in position 4 are protected as benzyl ether avoiding the problem of hydrogenolysis of the C-O bond in the same position (-O-Bn is stable when hydrogenation is carried out in basic conditions).

The invention is further illustrated by the following examples.

### EXAMPLES

### Example 1- Preparation of dioxolanone (XIII)

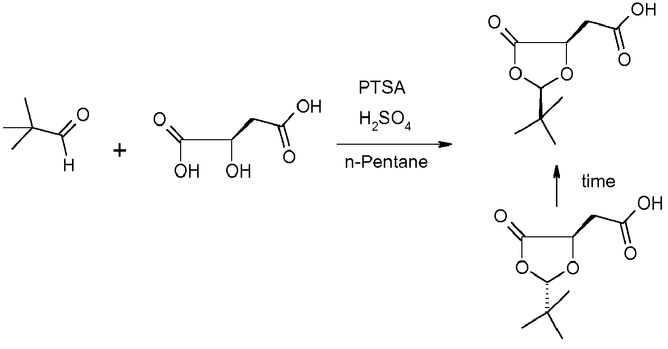

Pivalaldehyde (25 ml, 19.5 g, MW 86.13, 226.4 mmol, 1.1 eq) was dissolved in n-Pentane (270 ml). D-(+)-malic acid (27.6 g, MW 134.08, 205.82 mmol, 1 eq.), PTSA (3.9 g, MW 190, 20.6 mmol, 0.1 eq.) and H₂SO₄ (98 %, 0.2 ml) were added. The reaction mixture was heated at 48-53 °C under stirring for 72 hours removing water with a Dean-Stark apparatus. The reaction mixture was filtered, the residue solubilized in DCM (1L) and washed twice with aqueous H₃PO₄ (8 %, 2 x 100 ml) dried (Na₂SO₄) and evaporated till dryness (40 g). The residue was crystallized from DCM (120 mL) at - 22°C. Dioxolanone (XIII) was isolated as a white solid (33 g, MW 202.21, 163.2 mmol, reaction yield 79.3%).

### Example 2 - Preparation of 5-iodo-2-methyl-pent-3-yn-2-ol benzyl ether (XX)

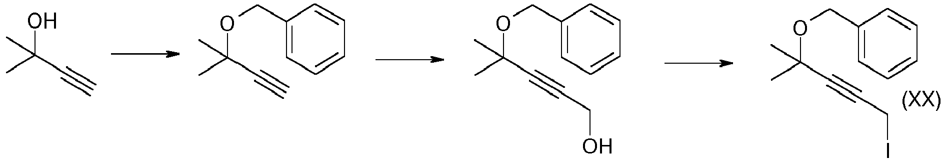

NaH (4.6 g, 60 % w/w in mineral oil, MW 24.00, 115 mmol, 1.05 eq.) was suspended in dry THF (100 ml) and cooled at 0°C under stirring. 3-hydroxy-3-methyl-1-butyne (9.2 g, MW 84.12, 109 mmol, 1 eq.) was dissolved in dry THF (10 ml) and slowly added into the reaction mixture. After addition the reaction temperature was let to raise at RT under stirring in 1 hour.

TBAI (369 mg, MW 369.37, 1 mmol, 0.01 eq.) was added and a solution of BnBr (18.6 g, MW 171.03, 109 mmol, 1 eq.) in dry THF (20 ml) was slowly added into the reaction mixture. The reaction was let to react for 6 hours and monitored by TLC (n-Hexane: EtOAc 95:5 stain: KMnO₄).

The reaction was quenched with water (200 ml) and extracted twice with n-Hexane (2 x 200 ml). The combined organic phases were evaporated till dryness yielding 3-hydroxy-3-methyl-1-butyne benzyl ether as a yellowish oil (20.1 g). The product was used in the next stage without further purifications.

3-hydroxy-3-methyl-1-butyne benzyl ether (20.1 g) was dissolved in THF dry (127 ml).

This solution was cooled at -25 °C and under vigorous stirring a solution of BuLi (2.5 M in n-hexane 50.7 ml) was added dropwise (50.7 ml/h). Para-formaldehyde (229.9 mmol, 6.89 g, 2 eq.) was added in portion into the reaction mixture (5 minutes) at -20 min and the temperature let to rise to RT (reaction is mild exothermic). After 6 hours the solution was cooled at 0 °C and quenched with aqueous 10 % NH₄Cl (200 ml) and NaHSO₄ solution to pH 6. The organic phase was separated and the aqueous one washed twice with DCM (2 x 100 ml). The combined organic phases were dried (Na₂SO₄) and evaporated till dryness yielding the desired compound 4-benzyloxy-4-methylpent-2-yn-1-ol (19 g, 204.78 MW, 84.6 %).

Iodine (5.9 g, MW 253.81, 102 mmol, 1.1 eq.) was dissolved in DCM (200 ml). PPh₃ (MW 262.29, 102.3 mmol, 26.8 g, 1.1 eq.) was added portionwise into the reaction mixture under vigorous stirring at 0°C (solution from deep purple turns to brown in 5 minutes), then imidazole (68.08 MW, 111.6 mmol, 7.59 g, 1.2 eq.) was added into the reaction mixture and let to react for 30 minutes. The temperature was let to rise at RT. 4-benzyloxy-4-methylpent-2-yn-1-ol (19 g, MW 204.27, 93 mmol) was dissolved in dry DCM (200 ml), added into the reaction mixture and stirred for 6 hours at RT (reaction was complete after warming the mixture at 30 °C for 30 min). TLC (n-hexane: EtOAc 9:1 Stain: KMnO₄). The reaction was kept under stirring at RT overnight.

The organic phase was washed once with water (1 x 200 ml) once with aqueous Na₂S₂O₃ (26 g in 200 ml + 1M KHSO₄ 20 ml) and then twice with Brine (2 x 200 ml). The organic phase was evaporated till dryness. The residue was triturated with n-hexane (200 ml) at 0°C. The suspension was filtered and the residue washed with cold n-hexane (100 ml). The combined organic phases were evaporated till dryness yielding compound (XX) (16.8 g, MW 314.86, 53.3 mmol, overall yield 48.8 %).
¹H NMR 400 MHz (CDCl₃) (δ ppm, J Hz): 7.26-7.48 (m, 5H, Ph), 4.61 (s, 2H, CH₂-Ph), 3.74 (s, 2H, CH₂-I), 1.51 (6H, s, CH₃)

### Example 3 - Preparation of 2-((2R,4R)-4-(4-(benzyloxy)- 4-methylpent-2-yn-1-yl)-2-(tert-butyl) -5-oxo-1,3-dioxolan-4-yl)acetic acid (XIX)

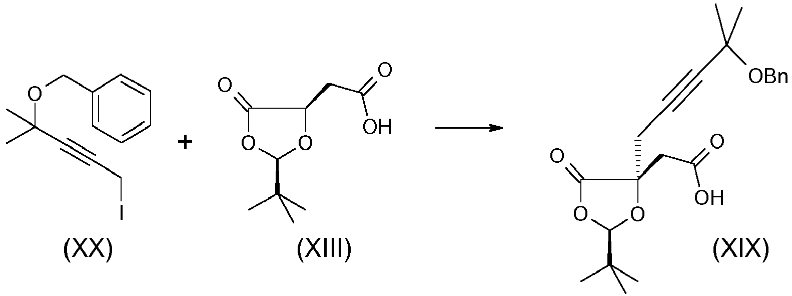

### Method A

Dioxolanone (XIII) (9 g, MW 202.21, 44.5 mmol) and compound (XX) (13.5 g, MW 314.17, 42.9 mmol) were dissolved in dry THF (400 ml). The solution was cooled at -78°C under N₂ atmosphere. A solution of LiHMDS (1M in THF, 89 ml, 89 mmol, 2 eq.) was added dropwise into the reaction mixture and let to react for 22 hours.

Glacial acetic acid (10 ml) was added into the reaction mixture. NaHSO₄ 1M (50 ml) was added to the reaction mixture (pH 1).

The temperature was let to raise at RT and the organic phase was separated. The aqueous phase was extracted twice with EtOAc (2 x 100 ml). The combined organic phases were washed twice with brine (2 x 100 ml), dried (Na₂SO₄), evaporated till dryness and purified over silica gel (SiO₂-flash 15 parts on the crude, eluted with DCM : MeOH 100 : 1) yielding compound (XIX) (8.5 g, MW 388, 21.9 mmol, 49.2 % yield).
¹H NMR 400 MHz (CDCl₃) (δ ppm, J Hz): 7.26-7.48 (m, 5H, Ph), 5.44 (s, 1H, OCHO), 4.60 (s, 2H, CH₂-Ph), 2.92 (d, J = 2.9 Hz, 2H, CH₂CC) 2.86 (d, J*_{AB}* = 17.0, 1H, CH₂CO₂), 2.79 (d, J*_{AB}* = 17.0, 1H, CH₂CO₂), 1.52 (s, 6H, CH₃), 0.93 (s, 9H, CH₃).

### Method B

Dioxolanone (XIII) (3 g, MW 202.21, 14.8 mmol) was dissolved in dry THF (400 ml). The solution was cooled at -78°C under N₂ atmosphere. A solution of LiHMDS in THF (1M, 44.5 ml, 44.5 mmol, 1 eq.) was added dropwise into the reaction mixture, then after 15 minutes another equivalent of LiHMDS in THF (1M, 44.5 ml, 44.5 mmol, 1 eq.) was added. The mixture was let to react for 8 hours at -78°C. Compound (XX) (4.7 g, MW 314.17, 14.9 mmol) was dissolved in dry THF (30 ml), added dropwise into the reaction mixture and let to react for 22 hours. Aqueous NH₄Cl (10 % w/v, 30 ml) was added dropwise into the reaction mixture, then NaHSO₄ 1M (80 ml) to reach pH 1. The temperature was let to raise at RT and the organic phase was separated. The aqueous phase was extracted twice with EtOAc (2 x 100 ml). The combined organic phases were washed twice with brine (2 x 100 ml), dried (Na₂SO₄), evaporated till dryness and purified over silica gel (SiO₂-flash 15 parts on the crude, eluted with DCM : MeOH 100 : 1). The product obtained (2.91 g, MW 388, 7.5 mmol, 50.6 % yield) showed identical characteristics to that of method A.

### Example 4 - Preparation of (R)-2-(4-(benzyloxy)-4-methylpent-2-yn-1-yl)-2-hydroxysuccinic acid (XXI)

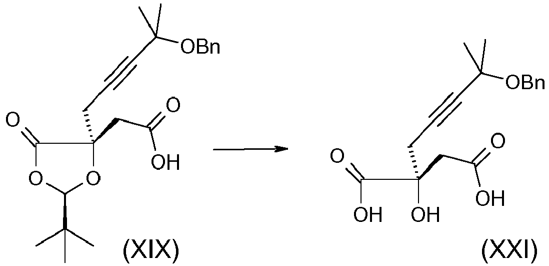

Compound (XIX) (100 mg, MW 388, 0.25 mmol, 1 eq.) was dissolved in a solution of KOH in MeOH (2M, 10 ml, 20 mmol, 10 eq.) under stirring at RT and left overnight. The solution was acidified with NaHSO₄ 1M (30 ml, to pH = 1) and extracted twice with DCM (2 x 20 ml). The combined organic phases were dried (Na₂SO₄) and evaporated till dryness yielding (XXI) (82 mg, MW 320, 0.24 mmol, 96 % yield).
¹H NMR 400 MHz (CDCl₃) (δ ppm, J Hz): 7.4-7.2 (5H, m, Ph); 4.6 (2H, s, CH₂-Ph); 3.05 (1H, d, J*_{AB}* = 16.8, CH₂CO₂); 2.85 (1H, d, J*_{AB}* = 16.6, CH₂CO₂); 2.65 (2H, dd, J*_{AB}* = 19.5; 16.6, CH₂CC); 1.51 (6H, CH₃)

### Example 5 - Preparation of (R)-dimethyl 2-(4-(benzyloxy)-4-methylpent-2-yn-1-yl)-2-hydroxysuccinate (XXII)

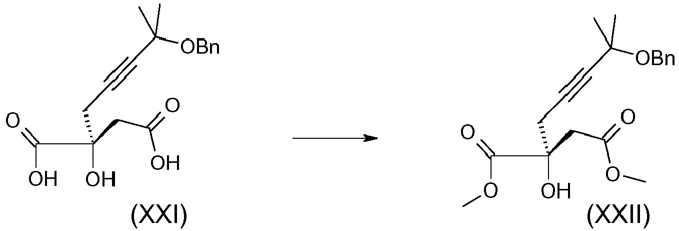

Compound (XXI) (2.75 g, MW 305.31, 9 mmol) was solubilized in a solution of BF₃ in MeOH (25 ml 12 % w/v) and the reaction solution was let to react under stirring at RT for 20 hours. The solution was quenched with saturated NaHCO₃ (pH 8), then extracted twice with DCM (2 x 50 ml). The combined organic phases were washed twice with Brine (2 x 20 ml) and evaporated till dryness. The product was purified over SiO₂-Flash (eluent n-Hexane : AcOEt 20:1). Product (XXII) showed the following characteristics:
¹H NMR 400 MHz (CDCl₃) (δ ppm, J Hz): 7.4-7.2 (5H, m, Ph); 4.6 (2H, s, CH₂-Ph); 3.9 (1H, s, OH); 3.8 (3H, s, OCH₃); 3.7 (3H, s, OCH₃); 3.0 (1H, d, J*_{AB}* = 16.2, CH₂CO₂); 2.8 (1H, d, J*_{AB}* = 16.2, CH₂CO₂); 2.67 (2H, s, CH₂CC); 1.54 (3H, s, CH₃); 1.51 (6H, CH₃)

### Example 6 - Preparation of (R)-dimethyl 2-(4-(benzyloxy)-4-methylpentyl)-2-hydroxysuccinate (XXIII)

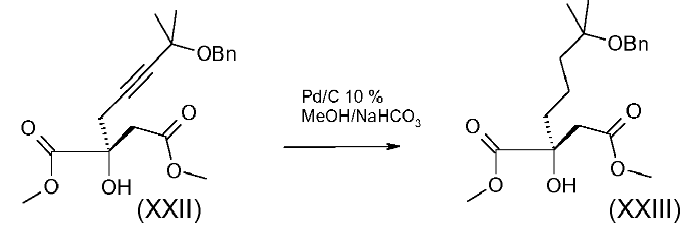

Compound (XXII) (1 g, MW 348.40, 2.8 mmol) was dissolved in a solution of NaHCO₃ in MeOH (final concentration 0.035 M). The solution was continuously hydrogenated (flow of 1 ml/min) over Pd/C 10% (150 mg) at 65 °C and under a H₂ pressure of 30 bar. The reaction solution was diluted with DCM, washed with NaHSO₄ 1M, dried (Na₂SO₄) and evaporated till dryness. The product obtained showed the following characteristics:
¹H NMR 400 MHz (CDCl₃) (δ ppm, J Hz): 7.4-7.2 (5H, m, Ph); 4.38 (2H, s, CH₂-Ph); 3.71 (1H, s, OH); 3.76 (3H, s, OCH₃); 3.67 (3H, s, OCH₃); 3.67 (1H, d, J*_{AB}* = 16.2, CH₂CO₂); 2.92 (1H, d, J*_{AB}* = 16.2, CH₂CO₂); 1.78-1.46 (2H, s, CH₂CC); 1.23 (3H, s, CH₃); 1.22 (6H, CH₃)

### Example 7 - Preparation of (R)-7-(benzyloxy)-3-hydroxy-3-(methoxycarbonyl)-7-methyloctanoic acid (XXVII)

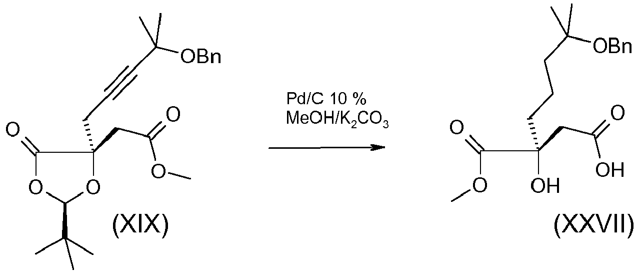

Compound (XIX) (250 mg, MW 388, 0.644 mmol) was dissolved in MeOH (2.5 ml), then K₂CO₃ (100 mg, MW 138.2, 0.723 mmol) and Pd/C (5% w/w wet, 50 mg) were added into the reaction mixture. The reaction vessel was purged with H₂ and the mixture was let to react at RT under vigorous stirring for 20 hours.

The suspension was diluted with MeOH (5 ml), filtered through a celite pad and evaporated till dryness. The residue was dissolved in KHSO₄ (1M, 3 mL to pH 1) and the product was extracted three times with EtOAc (3 x 2 ml). The combined organic phases were dried (Na₂SO₄) and evaporated till dryness. The product obtained showed the following characteristics:
¹H NMR 400 MHz (CDCl₃) (δ ppm, J Hz): 7.4-7.2 (5H, m, Ph); 4.4 (2H, s, CH₂-Ph); 3.75 (3H, s, OCH); 2.94 (1H, d, J*_{AB}* = 16.4, CH₂CO₂); 2.72 (1H, d, J*_{AB}* = 16.6, CH₂CO₂); 1.78-1.45 (2H, m, CH₂); 1.36-1.19 (4H, m, CH₂); 1.23 (6H, s, CH₃)

### Example 8 - Preparation of (R)-3,7-dihydroxy-3-(methoxycarbonyl)-7-methyloctanoic acid (XXVIII)

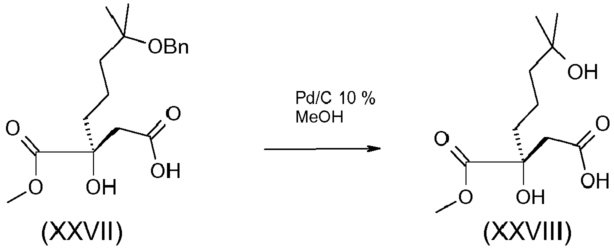

Compound (XXVII) (40 mg) was dissolved in MeOH (1 ml) and Pd/C (5%wet, 23 mg) was added into the reaction mixture.

After three cycles of vacuum/nitrogen the atmosphere was purged with H₂ at 1 atm and the reaction mixture was let to react at RT for 6 hours under vigorous stirring. The suspension was diluted with MeOH (5 ml), filtered through a celite pad and evaporated till dryness. The product obtained showed the following characteristics:
¹H NMR 400 MHz (CDCl₃) (δ ppm, J Hz): 4.39 (s-broad); 3.80 (3H, s, OCH₃); 2.95 (1H, d, J*_{AB}* = 16.4, CH₂CO₂); 2.73 (1H, d, J*_{AB}* = 16.4, CH₂CO₂); 1.80-1.37 (2H, m, CH₂); 1.30-1.08 (4H, m, CH₂); 1.2 (6H, s, CH₃)

### Example 9 - Preparation of (R)-dimethyl 2-(4-(benzyloxy)-4-methylpentyl)-2-hydroxysuccinate (XXIV)

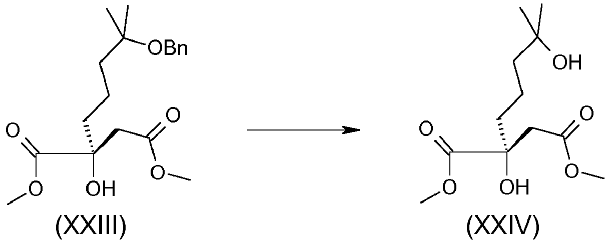

### Method A

Compound (XXIII) (1 g, MW 352.40, 2.83 mmol 1 eq.) was dissolved in a solution of AcOH (2% in MeOH) to have a final concentration of 0.2 M. The solution was continuously hydrogenated (flow 1 ml/min) over Pd/C 10% (150 mg) at ambient temperature and pressure. The product (XXIV) obtained showed the following characteristics:
¹H NMR 400 MHz (CDCl₃) (δ ppm, J Hz): 3.80 (3H, s, OCH₃); 3.68 (3H, s, OCH₃); 2.92 (1H, d, J*_{AB}* = 16.2, CH₂CO₂); 2.73 (1H, d, J*_{AB}* = 16.2, CH₂CO₂); 1.80-1.37 (2H, m, CH₂); 1.30-1.08 (4H, m, CH₂); 1.20(6H, s, CH₃)

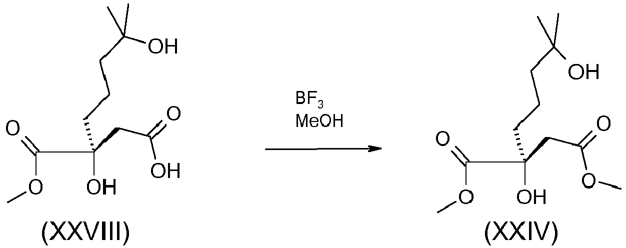

### Method B

Compound (XXVIII) (100 mg) was dissolved in a solution of BF₃ in MeOH (5 ml, 12 % w/v) and the reaction solution was let to react under stirring at RT for 20 hours. The solution was quenched with saturated NaHCO₃ (pH 8), then extracted twice with DCM (2 x 5 ml). The combined organic phases were washed twice with brine (2 x 2 ml) and evaporated till dryness. Product (XXIV) showed identical characteristics to that obtained with method A.

### Example 10 - Preparation of (R)-methyl 2-(2-methoxy-2-oxoethyl)-6,6-dimethyltetrahydro-2H-pyran-2-carboxylate (XXV)

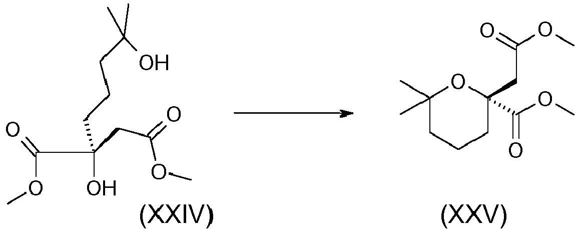

Compound (XXIV) (2.3 g, MW 244, 9.4 mmol, 1 eq) was dissolved in DCM (20 ml). Amberlyst-15-dry resin (1.74 g; ≥ 4.7 eq/Kg, 8.178 mmol active sites, 0.86 eq) was added and the suspension was stirred at RT for 20 hours. Resin was filtered off and washed three times with DCM (3 x 2 mL). The combined organic solutions were evaporated till dryness. Product (XXV) (2.1 g, MW 226, 9.4 mmol, quantitative yield) showed the following characteristics:
¹H NMR 400 MHz (CDCl₃) (δ ppm, J Hz): 3.74 (3H, s, OCH₃); 3.64 (3H, s, OCH₃); 2.84 (1H, d, J*_{AB}* = 14.2, CH₂CO₂); 2.60 (1H, d, J*_{AB}* = 14.2, CH₂CO₂); 2.30 (1H, m); 1.87 (1H, m); 1.61 (1H, m); 1.49 (2H, m); 1.42 (1H, m); 1.21 (3H , s, CH₃); 1.11 (3H, s, CH₃)

### Example 11 - Preparation of (R)-2-(carboxymethyl)-6,6-dimethyltetrahydro-2H-pyran-2-carboxylic acid (XXVI).

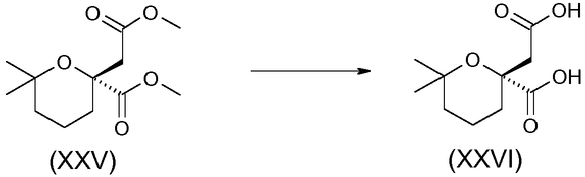

Potassium hydroxide (4.2 g, 75 mmol) in H₂O (50 ml) was added to a stirred solution of compound (XXV) (1.92 g, 7.5 mmol) in MeOH (70 ml) and the resulting solution was stirred at reflux for 5 hours. After cooling at room temperature, and removal of the methanol in vacuum, the solution was washed once with DCM (1 x 20 ml). The aqueous phase was acidified with KHSO₄ (1M to pH = 1) and extracted three times with EtOAc (3 x 30 ml). The combined organic layers were washed once with brine (1 x 20 ml), dried (Na₂SO₄) and evaporated to dryness to afford a pale yellow oil (1.66 g, 98%) that showed the following characteristics:
¹H NMR 400 MHz (CDCl₃) (δ ppm, J Hz): 3.00 (1H, d, J*_{AB}* = 16.1, CH₂CO₂); 2.98 (1H, d, J*_{AB}* = 16.1, CH₂CO₂); 1.89 (1H, m); 1.75 (2H, m, CH₂); 1.58 (3H, m); 1.31 (6H, s, CH₃).

### Example 12 - Preparation of (R)-2-(2-methoxy-2-oxoethyl)-6,6-dimethyltetrahydro-2H-pyran-2-carboxylic acid (I).

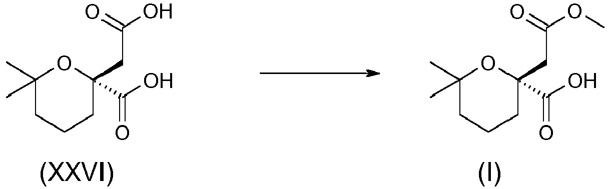

Compound (XXVI) (2.1 g, MW 216.24, 9.7 mmol) was dissolved in a solution of BF₃*MeOH (5 ml 12 % w/w freshly prepared) and stirred at 20 °C under vigorous stirring for 16 hours. The solution was cooled at 0°C and quenched with a saturated NaHCO₃ solution (30 ml). The solution was washed twice with diethyl ether (2 x 5 ml). The aqueous phase was acidified with NaHSO₄ (1M, 15 ml) to pH 1 and the product extracted with EtOAc (3 x 20 ml). The combined organic layers were dried (Na₂SO₄) and evaporated to dryness to afford a yellow oil (1.34 g, 60 %). The product obtained showed the following characteristics:
¹H NMR 400 MHz (CDCl₃) (δ ppm, J Hz): 3.70 (3H, s, CH₃O); 3.03 (1H, d, J*_{AB}* = 16.1, CH₂CO₂); 2.98 (1H, d, J*_{AB}* = 16.1, CH₂CO₂); 1.82 (1H, m); 1.74 (3H, m, CH₂); 1.62 (1H, m); 1.48 (1H, m); 1.31 (3H, s, CH₃); 1.26 (3H, s, CH₃).

## Claims

1. 2-((2*R*,4*R*)-4-(4-(benzyloxy)-4-methylpent-2-yn-1-yl)-2-(tert-butyl) -5-oxo-1,3-dioxolan-4-yl)acetic acid of formula (XIX).

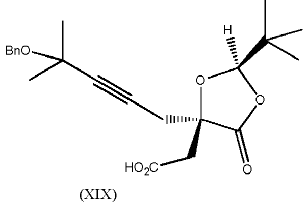

2. A process for the preparation of 2-((2R,4R)-4-(4-(benzyloxy)- 4-methylpent-2-yn-1-yl)-2-(tert-butyl) -5-oxo-1,3-dioxolan-4-yl)acetic acid of formula (XIX), consisting in the steps of treating compound 2-((2R,4R)-4-(4-(benzyloxy)- 4-methylpent-2-yn-1-yl)-2-(tert-butyl) -5-oxo-1,3-dioxolan-4-yl)acetic acid of formula (XIII) with a base and reacting the product of such reaction with 5-iodo-2-methyl-pent-3-yn-2-ol benzyl ether of formula (XX).

3. A process according to claim 2 where the base is a strong non-nucleophilic base.

4. A process according to claim 3 where the base is potassium-, lithium- or sodium-hexamethyldisilazane.

5. A process according to claim 3 where the base is lithium diispropylamide.

6. A process according to claim 2 where the base is added in the presence of both compound (XIII) and compound (XX).

7. A process for the conversion of compound (XIX) into (*R*)-2-(2-methoxy-2-oxoethyl)-6,6-dimethyltetrahydro-2*H*-pyran-2-carboxylic acid (I) 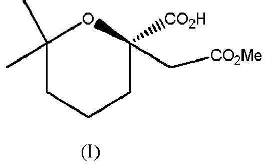 consisting in the steps of :
a) Hydrolysing 2-((2R,4R)-4-(4-(benzyloxy)- 4-methylpent-2-yn-1-yl)-2-(tert-butyl) -5-oxo-1,3-dioxolan-4-yl)acetic acid of formula (XIX) to produce (*R*)-2-(4-(benzyloxy)-4-methylpent-2-yn-1-yl)-2-hydroxysuccinic acid of formula (XXI); 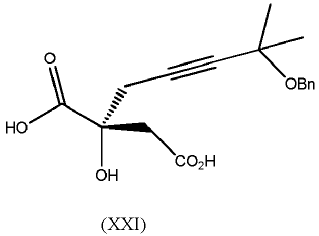
b) Esterifying (*R*)-2-(4-(benzyloxy)-4-methylpent-2-yn-1-yl)-2-hydroxysuccinic acid of formula (XXI) to produce (*R*)-dimethyl 2-(4-(benzyloxy)-4-methylpent-2-yn-1-yl)-2-hydroxysuccinate (XXII); 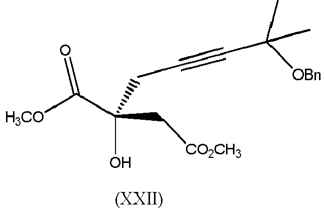
c) Hydrogenating in slightly basic conditions (R)-dimethyl 2-(4-(benzyloxy)-4-methylpent-2-yn-1-yl)-2-hydroxysuccinate of formula (XXII) to produce (R)-dimethyl 2-(4-(benzyloxy)-4-methylpentyl)-2-hydroxysuccinate of formula (XXIII); 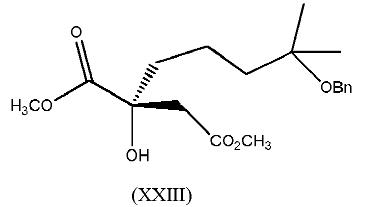
d) Hydrogenating in slightly acidic conditions (*R*)-dimethyl 2-(4-(benzyloxy)-4-methylpentyl)-2-hydroxysuccinate of formula (XXIII) to produce (R)-dimethyl 2-(4-(hydroxy)-4-methylpentyl)-2-hydroxysuccinate of formula (XXIV); 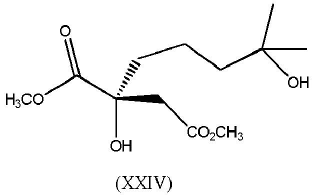
e) Cyclising in acidic conditions (*R*)-dimethyl 2-(4-(hydroxy)-4-methylpentyl)-2-hydroxysuccinate of formula (XXIV) to produce (*R*)-methyl 2-(2-methoxy-2-oxoethyl)-6,6-dimethyltetrahydro-2*H*-pyran-2-carboxylate of formula (XXV); 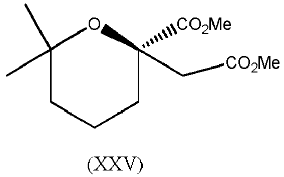
f) Hydrolysing (R)-methyl 2-(2-methoxy-2-oxoethyl)-6,6-dimethyltetrahydro-2*H-*pyran-2-carboxylate of formula (XXV) to produce (*R*)-2-(carboxymethyl)-6,6-dimethyltetrahydro-2*H*-pyran-2-carboxylic acid of formula (XXVI); 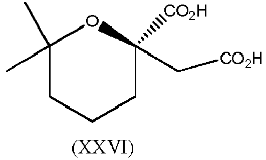
g) Monoesterifying (*R*)-2-(carboxymethyl)-6,6-dimethyltetrahydro-2*H-*pyran-2-carboxylic acid of formula (XXVI) to produce (*R*)-2-(2-methoxy-2-oxoethyl)-6,6-dimethyltetrahydro-2*H*-pyran-2-carboxylic acid of formula (I).

8. A process for the conversion of compound (XIX) into (*R*)-2-(2-methoxy-2-oxoethyl)-6,6-dimethyltetrahydro-2*H*-pyran-2-carboxylic acid (I) 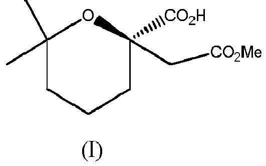 consisting in the steps of :
a) Hydrogenating in slightly basic conditions 2-((2*R*,4*R*)-4-(4-(benzyloxy)- 4-methylpent-2-yn-1-yl)-2-(tert-butyl) -5-oxo-1,3-dioxolan-4-yl)acetic acid of formula (XIX) to produce (*R*)-7-(benzyloxy)-3-hydroxy-3-(methoxycarbonyl)-7-methyloctanoic acid of formula (XXVII); 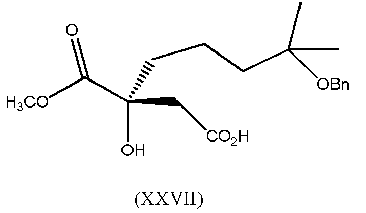
b) Hydrogenating (*R*)-7-(benzyloxy)-3-hydroxy-3-(methoxycarbonyl)-7-methyloctanoic acid (XXVII) to yield (R)-3,7-dihydroxy-3-(methoxycarbonyl)-7-methyloctanoic acid (XXVIII); 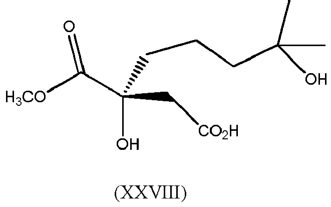
c) Esterification of (*R*)-3,7-dihydroxy-3-(methoxycarbonyl)-7-methyloctanoic acid (XXVIII) to yield (*R*)-dimethyl 2-(4-hydroxy-4-methylpentyl)-2-hydroxysuccinate (XXIV); 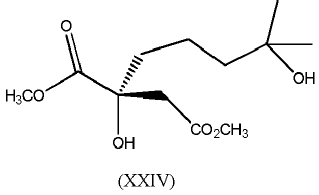
d) Cyclising in acidic conditions (R)-dimethyl 2-(4-hydroxy-4-methylpentyl)-2-hydroxysuccinate of formula (XXIV) to produce (*R*)-methyl 2-(2-methoxy-2-oxoethyl)-6,6-dimethyltetrahydro-2*H*-pyran-2-carboxylate of formula (XXV); 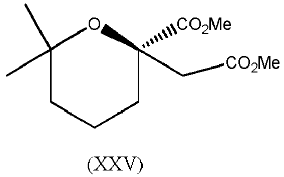
e) Hydrolysing (*R*)-methyl 2-(2-methoxy-2-oxoethyl)-6,6-dimethyltetrahydro-2*H-*pyran-2-carboxylate of formula (XXV) to produce (R)-2-(carboxymethyl)-6,6-dimethyltetrahydro-2*H*-pyran-2-carboxylic acid of formula (XXVI); 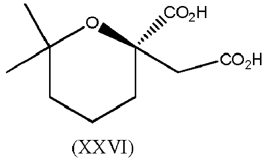
f) Monoesterifying (*R*)-2-(carboxymethyl)-6,6-dimethyltetrahydro-2*H-*pyran-2-carboxylic acid of formula (XXVI) to produce (*R*)-2-(2-methoxy-2-oxoethyl)-6,6-dimethyltetrahydro-2*H*-pyran-2-carboxylic acid (I).

9. A process according to claim 7 wherein in step b) the hydrogenation of (*R*)-7-(benzyloxy)-3-hydroxy-3-(methoxycarbonyl)-7-methyloctanoic acid (XXVII) is performed in methanol in slightly acid conditions to yield compound (XXIV), and step c) is omitted.

10. A compound selected from:
(*R*)-2-(4-(benzyloxy)-4-methylpent-2-yn-1-yl)-2-hydroxysuccinic acid of formula (XXI) 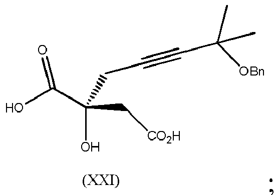
(R)-dimethyl 2-(4-(benzyloxy)-4-methylpent-2-yn-1-yl)-2-hydroxysuccinate of formula (XXII) 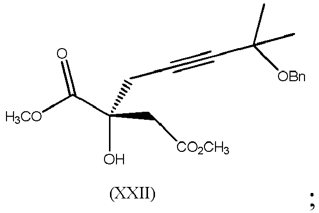
(*R*)-dimethyl 2-(4-(benzyloxy)-4-methylpentyl)-2-hydroxysuccinate of formula (XXIII) 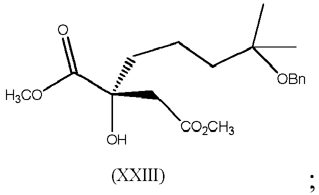
(*R*)-7-(benzyloxy)-3-hydroxy-3-(methoxycarbonyl)-7-methyloctanoic acid of formula (XXVII) 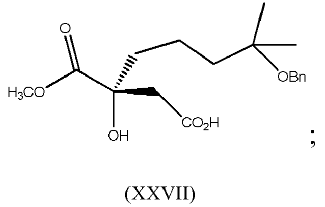
(*R*)-3,7-dihydroxy-3-(methoxycarbonyl)-7-methyloctanoic acid of formula (XXVIII)
